# EUROPEAN PATENT APPLICATION

(11) **EP 4 825 316 A1**
(43) Date of publication of application: **21.10.2026**
(21) Application number: 25170275.9
(22) Date of filing: 14.04.2025
(51) Int. Cl.: A61B 5/308, A61B 5/349, A61B 5/00

(54) **IDENTIFYING NOISY ELECTRODES**

(71) Applicant: Koninklijke Philips N.V., 5656 AE Eindhoven (NL)
(72) Inventor: BONOMI, Alberto Giovanni, 5656 AG Eindhoven (NL); HELFENBEIN, Eric, 5656 AG Eindhoven (NL); HUSEN, Sri Andari, 5656 AG Eindhoven (NL)
(74) Representative: Philips Intellectual Property & Standards

(57) **Abstract**

Proposed concepts thus aim to provide schemes, solutions, concept, designs, methods and systems pertaining to identifying noisy electrodes of an ECG measurement system. In particular, embodiments aim to provide a method for identifying noisy electrodes of an ECG measurement system by analyzing multiple ECG lead signals, and then, using a known correspondence between each lead signal and the pair of electrodes which generated it, identifying a noisy electrode based on noise indicator values of the lead signals. By identifying a noisy electrode, rather than merely identifying a noisy lead signal, said electrode can be directly dealt with (e.g., repositioned, replaced, or discarded), facilitating a more effective use of the ECG monitoring system.

## Description

### FIELD OF THE INVENTION

This invention relates to the field of identifying noisy electrodes of an ECG measurement system.

### BACKGROUND OF THE INVENTION

In real-time ECG monitoring, ECG signals are obtained through multiple electrodes attached to the body at several positions. The voltage difference measured between two of the electrodes is used to determine the bipolar lead ECG signals. Multiple bipolar leads are further derived from the electrodes, the signals of which are used for the real-time monitoring. For instance, the following Table shows how lead signals are derived from electrodes in a typical 5-wire standard cable setup:

**Table I**

| Lead | Electrode 1 (+) | Electrode 2 (-) |
|---|---|---|
| I | Left Arm (LA) | Right Arm (RA) |
| II | Left Leg (LL) | RA |
| III | LL | LA |
| CR (computed) | Chest (C) | RA |
| CL (computed) | C | LA |
| CF (computed) | C | LL |

Leads I, II, and III are standard limb leads which are bipolar leads. Leads CR, CL, CF are computed leads which are unipolar loads with central terminal reference. As bipolar leads are derived from the electrodes, disturbances on an electrode will impact the leads from which they are derived. Disturbance could arise from movement on the body part to which the electrode is attached, loose electrode contact with the skin, or other artifacts. Disturbances or noise may impact the ECG signal quality which subsequently may impact the quality of an ECG alarm raised by the monitor.

Providing timely interventions to patients suffering from critical arrhythmia is essential to improve patient outcomes in the ICU. Critical arrhythmias like ventricular tachycardia, ventricular fibrillation, asystole and extreme brady- and tachy-cardia can be automatically detected by algorithms capable of processing and interpreting electrocardiographic (ECG) recordings via, for example, bedside monitors.

Highly sensitive detection of these critical arrhythmias often results in numerous false alarms which have been reported to negatively impact clinical workload and worsen patient wellbeing. False alarms can be caused by noisy and weak ECG signals which may be caused by patient movement, electrode displacement, and other electrical interference during monitoring.

### SUMMARY OF THE INVENTION

The invention is defined by the claims.

According to examples in accordance with an aspect of the invention, there is provided a computer-implemented method for identifying noisy electrodes of an ECG measurement system.

The method comprises: determining a noise indicator value for each of a plurality of ECG lead signals, each ECG lead signal generated by a respective pair of electrodes of an ECG measurement system during an ECG measurement of a subject; analyzing the determined noise indicator values for each of the plurality of ECG lead signals in combination with a correspondence between each ECG lead signal and its respective pair of electrodes; and identifying at least one noisy electrode based on the result of the analysis.

Proposed concepts thus aim to provide schemes, solutions, concepts, designs, methods and systems pertaining to identifying noisy electrodes of an ECG measurement system. In particular, embodiments aim to provide a method for identifying noisy electrodes of an ECG measurement system by analyzing multiple ECG lead signals, and then, using a known correspondence between each lead signal and the different pair of electrodes which generated it, identifying a noisy electrode based on noise indicator values of the lead signals.

By identifying a noisy electrode, rather than merely identifying a noisy lead signal, said electrode can be directly dealt with (e.g., repositioned, replaced, or discarded), facilitating a more effective use of the ECG monitoring system. For instance, rather than having to entirely discard an identified noisy ECG lead signal, instead, the noise may be easily fixed by identifying the noisy electrode itself and, for example, conveying this information to a user such that they can reposition it (e.g., making sure it has better contact with the skin). Furthermore, identifying a noisy electrode may allow all ECG lead signals generated by said electrode to be discarded or de-prioritized in the lead selection process (e.g., for use in arrythmia detection), rather than having to, for example, decide lead-by-lead.

In summary, by leveraging the known correlation between an electrode and the ECG lead signals it is part of generating, noisy lead signals can be used to identify the noisy electrode (e.g., through covariance analysis) which is the source of said noise, thus allowing more effective interventions to fix said noise or to discard/deprioritize any ECG lead signals stemming from said noisy electrode.

Ultimately, an improved method for identifying noisy electrodes of an ECG measurement system.

In some embodiments, analyzing the determined noise indicator values may comprise determining a noise indicator value for each of the electrodes based on the noise indicator values of each of the plurality of ECG lead signals partially generated by said electrode; and identifying the at least one noisy electrode may comprise identifying an electrode as a noisy electrode if its determined noise indicator value is above a predetermined threshold. This provides an effective and/or efficient way to identify the at least one noisy electrode based on the noise indicator values for the ECG lead signals.

In some embodiments, determining a noise indicator value for an electrode may comprise averaging the noise indicator values of each of the plurality of ECG lead signals partially generated by said electrode. This provides an efficient way to determine a noise indicator value for an electrode.

In some embodiments, the noise indicator value may comprise a co-variance value of the ECG lead signal with another of the plurality of ECG lead signals. Co-variance provides an effective measure for identifying a noisy electrode based on ECG lead signals.

In some embodiments, the noise indicator value may comprise at least one of: high frequency noise indicator value; and a baseline wander value. These are effective measures of noise in an ECG lead signal.

In some embodiments, prior to determining a noise indicator value for each of a plurality of ECG lead signals, the method may comprise: detecting a position of a QRS complex in at least one of the plurality of ECG lead signals; and processing each of the plurality of ECG lead signals based on the at least one detected position of a QRS complex. This allows the noise indicator value to be more effectively determined.

In some embodiments, processing each of the plurality of ECG lead signals may comprise masking a portion of each ECG lead signal based on the at least one detected position of a QRS complex. This allows the strong components of the QRS complex to be removed from the signal, thus allowing noise to be more easily detected.

In some embodiments, detecting a position of a QRS complex in at least one of the plurality of ECG lead signals may comprise detecting a position of a QRS complex in each of the plurality of ECG lead signals; and processing each of the plurality of ECG lead signals may comprise masking out the detected QRS complex in each of the plurality of ECG lead signals respectively. This may ensure that the QRS complex is correctly masked out in each ECG lead signal.

In some embodiments, prior to determining a noise indicator value for each of a plurality of ECG lead signals, the method may comprise: detecting a position of a p and/or t wave in at least one of the plurality of ECG lead signals; and processing each of the plurality of ECG lead signals based on the detected position of the at least one p and/or t wave. This allows these strong components to be removed from the signal, thus allowing noise to be more easily detected.

In some embodiments, determining a noise indicator value for each of a plurality of ECG lead signals may comprise: applying a transformation to each of the plurality of ECG lead signals using a dimensionality reduction algorithm; and determining a noise indicator value for each of the plurality of ECG lead signals based on a correspondence between different components of each of the transformed plurality of ECG lead signals. These techniques allow for separation of ECG components from the noise components which can thereby aid in identifying noisy electrodes.

In some embodiments, the dimensionality reduction algorithm may comprise at least one of: a singular value decomposition algorithm; and an independent component analysis algorithm. These are both effective dimensionality reduction algorithms.

In some embodiments, the method may further comprise generating a display control signal, the display control signal indicating the identified at least one noisy electrode. This may allow a user to be notified about the noisy electrode and therefore allow them to adjust or replace it, for example.

In some embodiments, the method may further comprise analyzing a subset of the plurality of ECG lead signals to detect arrhythmia, the subset of the plurality of ECG lead signals consisting of the ECG lead signals which are generated by a respective pair of electrodes not including the identified at least one noisy electrode. This allows for more reliable detection of arrhythmia.

According to another aspect of the invention, there is provided a computer program comprising code means for implementing the method of any herein disclosed method when said program is run on a processing system.

According to another aspect of the invention, there is provided a system for identifying noisy electrodes of an ECG measurement system. The system comprising a processing arrangement configured to: determine a noise indicator value for each of a plurality of ECG lead signals, each ECG lead signal generated by a respective pair of electrodes of an ECG measurement system during an ECG measurement of a subject; analyze the determined noise indicator values for each of the plurality of ECG lead signals in combination with a correspondence between each ECG lead signal and its respective pair of electrodes; and identify at least one noisy electrode based on the result of the analysis.

Thus, there may be proposed concepts for identifying noisy electrodes of an ECG measurement system, and this may be done based on analyzing multiple ECG lead signals to determine noise indicator values and using a known correspondence between each lead signal and the pair of electrodes which generated it.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for identifying noisy electrodes of an ECG measurement system according to a proposed embodiment;
Fig. 2 is a flow diagram of a method for identifying noisy electrodes of an ECG measurement system according to a proposed embodiment;
Fig. 3 is a flow diagram of a method for identifying noisy electrodes of an ECG measurement system according to a proposed embodiment;
Fig. 4 is a flow diagram of a method for identifying noisy electrodes of an ECG measurement system according to a proposed embodiment;
Fig. 5 is a flow diagram of a method for identifying noisy electrodes of an ECG measurement system according to a proposed embodiment;
Fig. 6 is a simplified block diagram of a system for identifying noisy electrodes of an ECG measurement system according to a proposed embodiment; and
Fig. 7 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to identifying noisy electrodes of an ECG measurement system. According to proposed concepts, a number of possible solutions may be implemented separately or jointly. That is, although these possible solutions may be described below separately, two or more of these possible solutions may be implemented in one combination or another.

Embodiments of the invention aim to provide a method for identifying noisy electrodes of an ECG measurement system. This can be achieved by analyzing multiple ECG lead signals, and then, using a known correspondence between each lead signal and the pair of electrodes which generated it, identifying a noisy electrode based on noise indicator values of the lead signals.

By identifying a noisy electrode, rather than merely identifying a noisy lead signal, said electrode can be directly dealt with (e.g., repositioned, replaced, or discarded), facilitating a more effective use of the ECG monitoring system. For instance, rather than having to entirely discard an identified noisy ECG lead signal, instead, the noise may be easily fixed by identifying the noisy electrode itself and repositioning it (e.g., making sure it has better contact with the skin). Furthermore, identifying a noisy electrode may allow all ECG lead signals generated by said electrode to be discarded or de-prioritized in a lead selection process, rather than having to, for example, decide lead-by-lead.

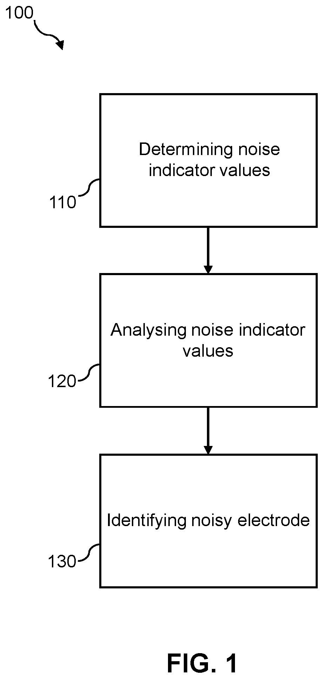

Referring now to Fig. 1, there is depicted a simplified flow diagram of a method 100 for identifying noisy electrodes of an ECG measurement system according to a proposed embodiment.

The method 100 begins with the step 110 of determining a noise indicator value for each of a plurality of ECG lead signals, each ECG lead signal generated by a (different) respective pair of electrodes of an ECG measurement system during an ECG measurement of a subject (i.e., the ECG measurement system being employed for measurement of a subject). As the skilled person would understand, an ECG lead signal is an electrical signal recorded using a pair of electrodes placed in different positions on a subject (as exemplified in Table I) to measure the electrical potential difference between those points on the subject's body. As the skilled person would appreciate, an ECG measurement system typically includes electrodes, lead wires connecting electrodes to a main recording unit (with amplifiers and filters), and a display.

A noise indicator value can be understood as a (numerical) metric that quantifies the amount of interference or unwanted signal variations in the ECG lead signal. In this embodiment, the noise indicator value comprises a co-variance value of the ECG lead signal with another of the plurality of ECG lead signals. Co-variance provides an effective measure for identifying a noisy electrode based on ECG lead signals. Covariance between ECG leads measures how the two signals vary together over time. It's calculated by finding how much each signal deviates from its average value at each moment, multiplying these deviations, and taking their average across the recording period. A positive covariance indicates the signals tend to increase and decrease together, while negative means they move in opposite directions. Covariance between ECG leads helps identify noise because true cardiac signals typically have consistent relationships between leads, while noise tends to be more random. When noise contaminates signals, the normal covariance patterns between leads are disrupted, making this metric useful for detecting signal quality issues. Of course, as the skilled person would appreciate, in some embodiments, for each ECG lead signal, a co-variance value is calculated for its pairing with each of the other ECG lead signals (i.e., resulting in multiple co-variance values per ECG lead signal).

In this embodiment, the noise indicator value further comprises a high frequency noise indicator value. In other words, in this embodiment, two noise indicator values are determined for each of the plurality of ECG lead signals: a co-variance value (which may itself include multiple values, as described above) and a high frequency noise indicator value. A high frequency noise indicator value measures rapid signal fluctuations in an ECG lead by analyzing components above a certain frequency threshold, typically higher than normal cardiac electrical activity. This is useful for identifying interference from noisy electrodes.

In some embodiments, the noise indicator value can alternatively/additionally comprise a baseline wander value. A baseline wander value measures low-frequency signal drift in an ECG lead. It quantifies how much the signal's baseline deviates from zero over time. Baseline wander detection is valuable for noise assessment because clean ECG signals should maintain a relatively sTable baseline. Significant wandering indicates poor signal quality, often from patient movement or electrode issues, making it a reliable indicator of recording problems.

Step 120 comprises analyzing the determined noise indicator values for each of the plurality of ECG lead signals in combination with a correspondence between each ECG lead signal and its respective pair of electrodes. Step 130 comprises identifying at least one noisy electrode based on the result of the analysis. For instance, as each ECG lead signal corresponds to a different pair of electrodes (i.e., the electrodes which generated the lead signal), by analyzing which ECG lead signals are noisy, it can be determined which electrodes must be noisy (i.e., the cause of the noise in the ECG lead signals). In a simple example, if all the ECG lead signals generated using a particular electrode are noisy but none of the other ECG lead signals are noisy, it can be determined that said particular electrode must be a noisy electrode.

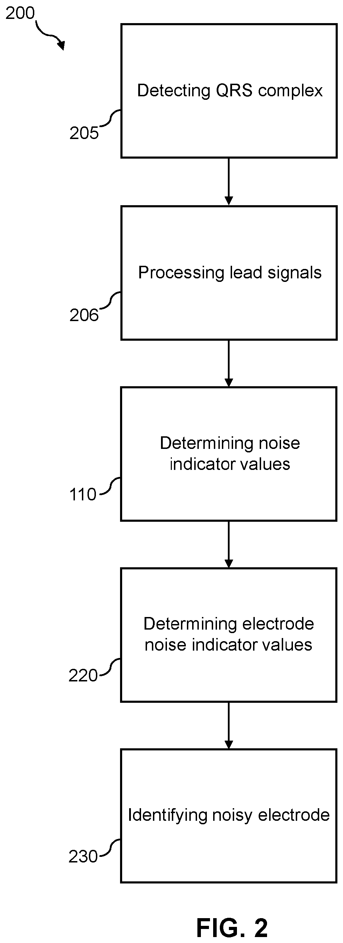

Referring now to Fig. 2, there is depicted a flow diagram of a method 200 for identifying noisy electrodes of an ECG measurement system according to a proposed embodiment. Step 110 is substantially the same as step 110 described in relation to method 100 of Fig. 1.

Step 205 comprises detecting a position of a QRS complex in at least one of the plurality of ECG lead signals. As the skilled person would know, a QRS complex is the part of an ECG lead signal that represents the rapid depolarization of the ventricles, leading to their contraction. Step 206 comprises processing each of the plurality of ECG lead signals based on the at least one detected position of a QRS complex. This allows the noise indicator value to be more effectively determined. For example, the position of the QRS complex can be detected in a single ECG lead signal and then this single position can be used to process each of the ECG lead signals. This may allow each of the ECG lead signals to be processed (e.g., adapted) accordingly even if their QRS complex is too weak to be reliably detected.

In this embodiment, processing each of the plurality of ECG lead signals in step 206 specifically comprises masking a portion of each ECG lead signal based on the at least one detected position of a QRS complex. This allows the strong components of the QRS complex to be removed from the signal, thus allowing noise to be more easily detected.

It should be noted that in some embodiments, step 205 can, in fact, comprise detecting a position of a QRS complex in each of the plurality of ECG lead signals. In these embodiments, step 206 then comprises masking out the detected QRS complex in each of the plurality of ECG lead signals respectively (i.e., using each ECG lead signal's respective detected position of the QRS complex). This can help ensure that the QRS complex is correctly masked out in each ECG lead signal. In some embodiments, however, after detecting a position of a QRS complex in each of the ECG lead signals in step 205, a single position can be generated using all the detected positions for use in subsequent masking across all of the ECG lead signals. For example, all the detected positions can be averaged into a single average position, or a majority voting system could be used.

Step 220 comprises determining a noise indicator value for each of the electrodes based on the noise indicator values of each of the plurality of ECG lead signals partially generated by said electrode. In other words, the noise indicator values of the ECG lead signals corresponding to each electrode (for example, as shown in Table I) are used to determine a noise indicator value for said electrode. For instance, in this embodiment, step 220 specifically comprises averaging the noise indicator values of each of the plurality of ECG lead signals partially generated by said electrode. This provides an efficient way to determine a noise indicator value for an electrode though, as the skilled person would appreciate, other suitable methods can also be used to determine a noise indicator value for each electrode using the noise indicator values of the corresponding ECG lead signals (e.g., a median, a mode, a maximum, a percentile-based combined value, etc.)

Step 230 accordingly comprises identifying an electrode as a noisy electrode if its determined noise indicator value is above a predetermined threshold. This provides an effective and/or efficient way to identify the at least one noisy electrode based on the noise indicator values for the ECG lead signals. The predetermined threshold can comprise an absolute value and/or a percentage value and, in some embodiments, is adjustable by a user. Furthermore, it should be noted that if the noise indicator values for the ECG lead signals comprise more than one type of value (e.g., high frequency noise indicator and co-variance), the noise indicator values for the electrodes can correspondingly comprise more than one type of value and, accordingly, different predetermined thresholds can be used for each type of value - an electrode can then be identified as noisy if at least one of (or alternatively, all of) the noise indicator values are above their respective predetermined thresholds.

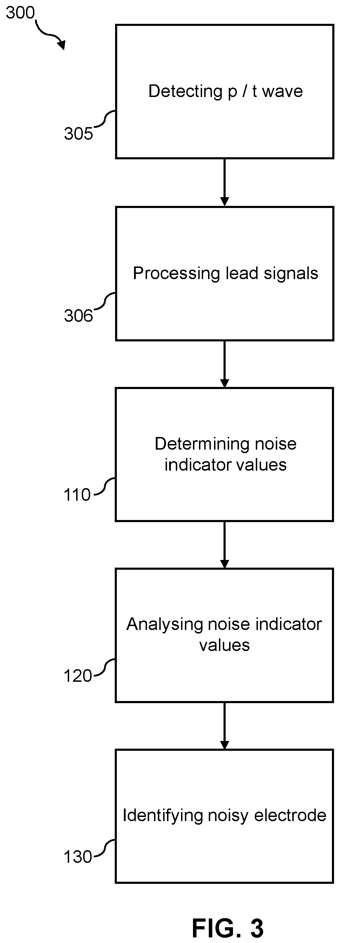

Referring now to Fig. 3, there is depicted a flow diagram of a method 300 for identifying noisy electrodes of an ECG measurement system according to a proposed embodiment. Steps 110, 120, and 130 are substantially the same as have been described in relation to the method 100 in Fig. 1.

Step 305 comprises detecting a position of a p and/or t wave in at least one of the plurality of ECG lead signals. Step 306 comprises processing each of the plurality of ECG lead signals based on the detected position of the at least one p and/or t wave. This allows these strong components to be removed from the signal, thus allowing noise to be more easily detected. In other words, steps 305 and 306 are substantially the same as steps 205 and 206 of method 200, however, using p and/or t waves in addition to QRS complex. Steps 305 and 306 can thus vary in the same ways as described above for steps 205 and 206, *mutatis mutandis.*

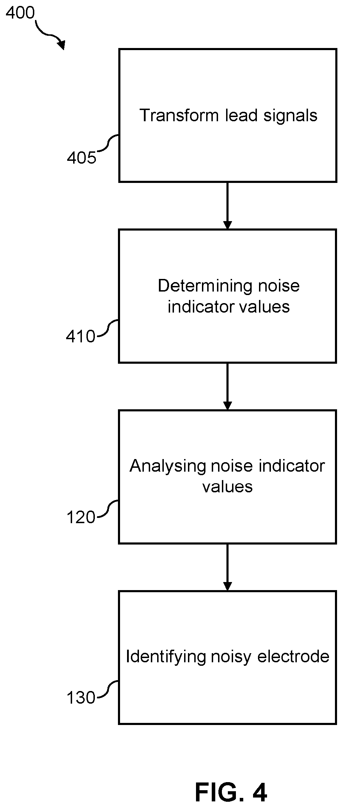

Referring now to Fig. 4, there is depicted a flow diagram of a method 400 for identifying noisy electrodes of an ECG measurement system according to a proposed embodiment. Steps 120 and 130 are substantially the same as have been described in relation to the method 100 in Fig. 1.

Step 405 comprises applying a transformation to each of the plurality of ECG lead signals using a dimensionality reduction algorithm. This reduces the complexity of the ECG lead signals by transforming them into a lower-dimensional representation while preserving relevant information. In this embodiment, the dimensionality reduction algorithm comprises at least one of: a singular value decomposition algorithm; and an independent component analysis algorithm. These are both effective dimensionality reduction algorithms, though as the skilled person would appreciate, in other embodiments, other suitable dimensionality reduction algorithms can be used. These techniques allow for separation of ECG components from the noise components which can thereby aid in identifying noisy electrodes.

Step 410 accordingly comprises determining a noise indicator value for each of the plurality of ECG lead signals based on a correspondence between different components of each of the transformed plurality of ECG lead signals. In other words, this step comprises analyzing the transformed ECG lead signals to identify relationships between corresponding components of different lead signals (e.g., between the noise components of different lead signals). In this way, for example, the ECG lead signals with the largest noise components can be more easily identified.

In a specific example of the invention, if a single electrode is noisy, this should be visible in the lead signals which are generated using said electrode, as shown in the following table, Table II (using the lead setup shown in Table I):

**Table II**

| Noisy Electrode | Noisy Leads | Clean Leads |
|---|---|---|
| RA | I, II, CR | III, CL, CF |
| LA | I, III, CL | II, CR, CF |
| LL | II, III, CF | I, CR, CL |
| C | CR, CL, CF | I, II, III |

In another specific example, if two electrodes are noisy, the noisy and clean lead signals will be as shown in the following table, Table III:

**Table III**

| Noisy Electrodes | Noisy Leads | Correlated Grouping | Clean Lead |
|---|---|---|---|
| RA, LA | I, II, III, CR, CL | (I, II, CR) ; (I, III, CL) | CF |
| RA, LL | I, II, III, CR, CF | (I, II, CR) ; (II, III, CF) | CL |
| RA, C | I, II, CR, CL, CF | (I, II, CR) ; (CR, CL, CF) | III |
| LA, LL | I, II, III, CL, CF | (I, III, CL) ; (II, III, CF) | CR |
| LA, C | I, III, CR, CL, CF | (I, III, CL) ; (CR, CL, CF) | II |
| LL, C | II, III, CR, CL, CF | (II, III, CF) ; (CR, CL, CF) | I |

The invention thus aims at detecting noisy electrodes using the lead signals. Specifically, noise indicators are derived from the lead signals, and these are then used to determine whether one or more electrodes of the ECG measurement system is noisy or not (e.g., reverse engineering the example situations shown in Tables II and III). The electrodes can then be, for example, repositioned, replaced, or discarded.

In a preferred embodiment of the invention, a pre-processing step can be used to enhance the performance of noise detection in lead signals.

In a specific example embodiment of the invention, a segment of a filtered ECG lead signal is pre-processed (to enhance the performance of noisy lead detection covariance calculation). As the ECG signal, especially the QRS complexes, are strong, they influence the detection of noisy leads as well as the covariance calculation. Masking the QRS complex will therefore ensure that the strong QRS components are excluded from the noise indicator value determination and/or the covariance calculation, allowing for the noise component to be more dominant.

The pre-processing step comprises the detection of QRS complexes and their positions. Once the positions of the complexes are found, the ECG signal at those positions is masked and used for further processing. The detection of the QRS complex and its position may be done on every lead independently, and further the masking of the QRS complex may also be done independently per lead. This may, however, result in variation of masked positions across the leads. To prevent that, the same position of the QRS complex can be used across all the leads. This can be done, for example, by pre-specifying a lead to be used for QRS complex detection, the derived position of which can be used for masking signals from all the leads. Alternatively, the QRS positions derived from all the leads can be combined, for example, by taking the conjunction of the positions across all the leads or using a majority voting procedure. The latter may help in case of a weak lead for which QRS complexes are difficult to detect. Another alternative is to combine the leads with the same polarity (e.g., positive polarity) and feed the combined signal into the QRS detector to derive a single set of positions of QRS complexes. As the skilled person would appreciate, there are many suitable ways to detect QRS complexes such as, for example, the Pan-Thomkins or Kethirvel algorithm.

After this pre-processing step (i.e., detecting and masking QRS complexes out of each ECG lead signal), two measures can be used as noise indicators for each ECG lead signal: a high frequency noise indicator and co-variance of a pair of leads. The high frequency noise indicator provides a measure to the strength of the high frequency noise while the co-variance measures the similarity of the variation in the signals of the pair of electrodes. There are other types of noise that may be present in an ECG signal, such as baseline wander, and for such noise, an additional noise measure can be used.

In an example, the high frequency noise can be detected as follows. For each segment (e.g., six seconds long) of masked ECG signal of a lead *l*, the difference between every two consecutive samples is calculated. The average of the absolute of this difference signal is used as an indicator for the noise strength of the segment. ${V}_{s , l} = \frac{1}{{N}_{s}} {\sum }_{i = 0}^{{N}_{s} - 1} \left|{x}_{{sN}_{s} + i + 1 , l}-{x}_{{sN}_{s} + i , l}\right|$ (1)

In equation (1), *x_{i,l}* is the sample *I* of masked ECG signal from lead *l* and *N_{S}* is the number of samples in a segment.

In another example, the high frequency noise can be detected as follows. This method is particularly suitable for detecting surges in the noise level. It uses the same indicator as before but normalizes it to the average of *M* previous values of the indicator. ${V}_{s , l}^{norm} = {V}_{s , l} / \left(\frac{1}{M}{\sum }_{k = 1}^{M} {V}_{s - k , l}\right)$ (2)

Co-variance is also calculated and is used to indicate how closely the masked ECG signal of a pair of leads varies. Co-variance between two leads is derived using sample Pearson correlation coefficient. For example: ${r}_{s} \left(l, k\right) = \frac{{\sum }_{i = 0}^{{N}_{s} - 1} \left({x}_{{sN}_{s} + i , l}-{\overline{x}}_{s , l}\right) \left({x}_{{sN}_{s} + i , k}-{\overline{x}}_{s , k}\right)}{\sqrt{{\sum }_{i = 0}^{{N}_{s} - 1} {\left({x}_{{sN}_{s} + i , l}-{\overline{x}}_{s , l}\right)}^{2}} \sqrt{{\sum }_{i = 0}^{{N}_{s} - 1} {\left({x}_{{sN}_{s} + i , k}-{\overline{x}}_{s , k}\right)}^{2}}}$ (3)

Where ${\overline{x}}_{s , l} = \frac{1}{M} {\sum }_{k = 1}^{{N}_{s} - 1} {x}_{{sN}_{s} + k , l}$ is the sample mean of segment s of the masked ECG signal of lead *l*.

Using the noise level strength (i.e., high frequency noise) and the co-variance, two electrode level indicators can be derived.

The average noise strength for electrode e: ${V}_{s , e} = \frac{1}{\left|{G}_{e}\right|} {\sum }_{l ∈ {G}_{e}} {V}_{s , l}$ (4)

Where *Gₑ* is the list of leads derived from electrode e (see, for example, column 'Noisy Leads' of Table II and Table III). The average noise strength per electrode may be generalized as follows: ${V}_{s , e} = {\sum }_{l ∈ {G}_{e}} {w}_{l} {V}_{s , l}$ (5)

Where ∑_{*l*∈}*_{Gₑ} wₗ =* 1.

The average of the absolute value of co-variance for electrode e can be given as: ${r}_{s , e} = {\sum }_{l \neq k , l , k ∈ {G}_{e}} {w}_{l , k} \left|{r}_{s}\left(l, k\right)\right|$ (6)

Where Σ_{*l*≠*k,l,k*∈}*_{Gₑ}w_{l,k}* = 1. An electrode is considered noisy if *V_{s,e}* > predetermined noise threshold and *r_{s,e}* > predetermined co-variance threshold. The thresholds are chosen such that they maximize the area under the ROC curve.

In addition to noise strength for the noisy lead grouping, the noise strength for the clean lead grouping may also be calculated, denoted as *V_{s,c}.* This noise strength can also be used to derive a certainty of noise on the noisy grouping. For instance, the relative noise strength of a candidate noisy electrode can be calculated as *V_{s,e}* / (*V_{s,e} + V_{s,c}*) or the noise strength ratio between the noisy grouping and the clean grouping can be derived as *V_{s,e}* / *V_{s,c}.* These certainty indicators can be used as an additional rule to the aforementioned rules (e.g., the thresholds) for identifying a noisy electrode, for instance, the relative noise strength may need to be at least 70% as well for the electrode to be identified / classified as noisy.

The two criteria (noise strength and covariance) can be calculated for every possible noisy electrode combination (e.g., as shown in Tables II and III). When multiple possible noisy electrodes arise, the certainty indicator can be used to choose the most likely noisy electrodes.

The certainty indicator can also be used to improve V-Tach (Ventricular Tachycardia) detection. For example, a potential V-Tach detection can be checked to see whether it may be explained purely by one or more noisy electrodes. If the probability is high (shown by the certainty indicator) that there is noise from one or two electrodes, for example, based on the noise distribution across the leads, a V-tach alarm may not be output. However, if it is unlikely that there is one or two noisy electrodes, then it is more likely that the V-tach detection is true.

In another example embodiment, the pre-processing aims at masking not only the QRS complex but also the *p* and *t* waves. Furthermore, in some embodiments, only co-variance can be used as a means to detect the noisy electrode with no dedicated indicator noise level. The reason for this is that if all information is removed from the ECG signal, all that remains will be noise, and if the noise comes from a particular electrode, a strong co-variance will indicate that.

In another example embodiment, rather than calculating the noise indicator per lead, the noise indicator can be derived on the combined lead signal, for example, according to the lead grouping shown in column 'Noisy Leads' of Tables II and III.

For example: ${y}_{i , e} = {\sum }_{l ∈ {G}_{e}} {b}_{l} {x}_{i , l}$ (7) ${V}_{s , e} = \frac{1}{{N}_{s}} {\sum }_{i = 0}^{{N}_{s} - 1} \left|{y}_{{sN}_{s} + i + 1 , l}-{y}_{{sN}_{s} + i , l}\right|$ (8)

In another example embodiment, instead of identifying and masking *QRS, p* and *t* waves from the ECG signal, prior to noise and co-variance detection, an alternative solution is to apply a transformation to the single lead ECG signal using dimensionality reduction techniques like singular value decomposition, and independent component analysis. These techniques allow for separation of ECG components from the noise components which can then be analyzed to measure correspondence between pairs of leads and thereby identify noisy electrodes.

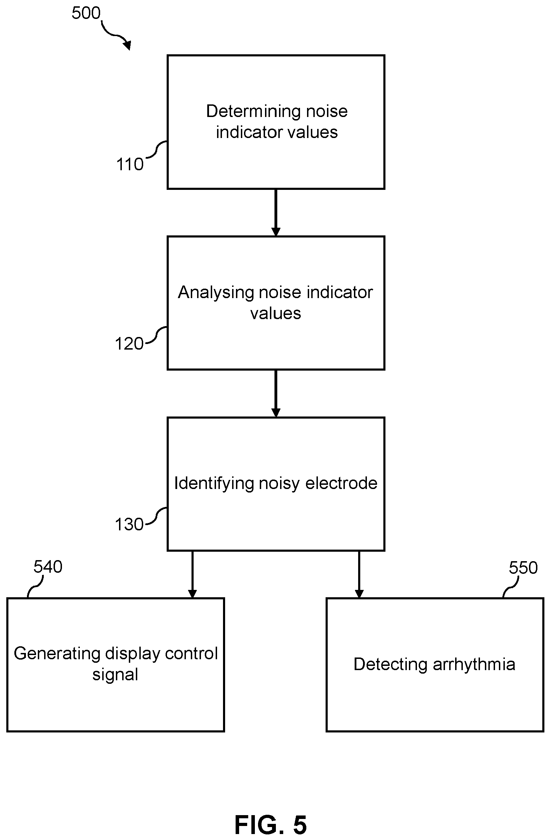

Referring now to Fig. 5, there is depicted a method 500 for identifying noisy electrodes of an ECG measurement system according to a proposed embodiment. Steps 110, 120, and 130 are substantially the same as have been described in relation to the method 100 in Fig. 1.

Step 540 comprises generating a display control signal, the display control signal indicating the identified at least one noisy electrode. This allows a user to be notified about the noisy electrode and therefore allow them to adjust its placement or replace it, for example. In some embodiments, the method 500 can actually include the step of outputting the generated display control signal to a display. For example, the display may be part of the ECG measurement system.

Step 550 comprises analyzing a subset of the plurality of ECG lead signals to detect arrhythmia (of the subject), the subset of the plurality of ECG lead signals consisting of the ECG lead signals which are generated by a respective pair of electrodes not including the identified at least one noisy electrode. In other words, the subset of the ECG lead signals comprises all the ECG lead signals minus those corresponding to identified noisy electrode(s) (i.e., only ECG lead signals exclusively generated by 'clean' non-noisy electrodes). This allows for more reliable detection of arrhythmia. In some embodiments, only one of steps 540 or 550 are performed.

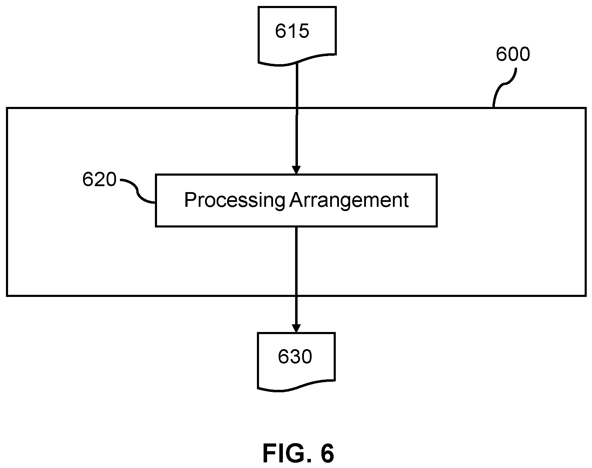

Referring now to Fig. 6, there is depicted a system 600 for identifying noisy electrodes of an ECG measurement system according to a proposed embodiment. The system 600 comprises a processing arrangement 620.

The processing arrangement 620 can be configured to perform any herein-disclosed method (e.g., any of methods 100, 200, 300, 400, or 500). In this embodiment specifically, however, the processing arrangement 620 is configured to perform method 100, i.e., to: determine a noise indicator value for each of a plurality of ECG lead signals, each ECG lead signal generated by a respective pair of electrodes of an ECG measurement system during an ECG measurement of a subject; analyze the determined noise indicator values for each of the plurality of ECG lead signals in combination with a correspondence between each ECG lead signal and its respective pair of electrodes; and identify at least one noisy electrode based on the result of the analysis. The input 615 into the system is thus a plurality of ECG lead signals and the output 630 of the system is an identification of at least one noisy electrode.

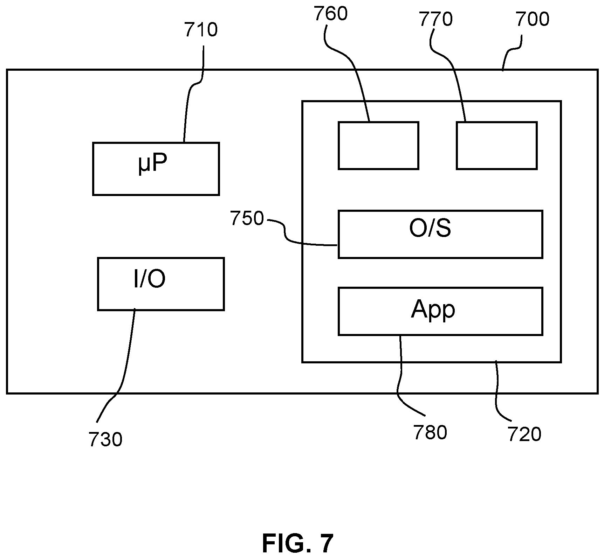

Fig. 7 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-5, and the system of Fig. 6, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for identifying noisy electrodes of an ECG measurement system, the method comprising:
determining a noise indicator value (110) for each of a plurality of ECG lead signals, each ECG lead signal generated by a respective pair of electrodes of an ECG measurement system during an ECG measurement of a subject;
analyzing the determined noise indicator values (120) for each of the plurality of ECG lead signals in combination with a correspondence between each ECG lead signal and its respective pair of electrodes; and
identifying at least one noisy electrode (130) based on the result of the analysis.

2. The computer-implemented method of claim 1, wherein:
analyzing the determined noise indicator values comprises determining a noise indicator value for each of the electrodes (220) based on the noise indicator values of each of the plurality of ECG lead signals partially generated by said electrode; and
identifying the at least one noisy electrode comprises identifying an electrode as a noisy electrode if its determined noise indicator value is above a predetermined threshold (230).

3. The computer-implemented method of claim 2, wherein determining a noise indicator value for an electrode (230) comprises averaging the noise indicator values of each of the plurality of ECG lead signals partially generated by said electrode.

4. The computer-implemented method of any of claims 1 to 3, wherein the noise indicator value comprises a co-variance value of the ECG lead signal with another of the plurality of ECG lead signals.

5. The computer-implemented method of any of claims 1 to 4, wherein the noise indicator value comprises at least one of: high frequency noise indicator value; and a baseline wander value.

6. The computer-implemented method of any of claims 1 to 5, wherein, prior to determining a noise indicator value for each of a plurality of ECG lead signals, the method comprises:
detecting a position of a QRS complex (205) in at least one of the plurality of ECG lead signals; and
processing each of the plurality of ECG lead signals (206) based on the at least one detected position of a QRS complex.

7. The computer-implemented method of claim 6, wherein processing each of the plurality of ECG lead signals (206) comprises masking a portion of each ECG lead signal based on the detected position of the at least one QRS complex.

8. The computer-implemented method of claim 7, wherein detecting a position of a QRS complex (205) in at least one of the plurality of ECG lead signals comprises detecting a position of a QRS complex in each of the plurality of ECG lead signals; and
processing each of the plurality of ECG lead signals (206) comprises masking out the detected QRS complex in each of the plurality of ECG lead signals respectively.

9. The computer-implemented method of any of claims 1 to 8, wherein prior to determining a noise indicator value for each of a plurality of ECG lead signals, the method comprises:
detecting a position of a p and/or t wave (305) in at least one of the plurality of ECG lead signals; and
processing each of the plurality of ECG lead signals (306) based on the detected position of the at least one p and/or t wave.

10. The computer-implemented method of any of claims 1 to 9, wherein determining a noise indicator value for each of a plurality of ECG lead signals comprises:
applying a transformation to each of the plurality of ECG lead signals (405) using a dimensionality reduction algorithm; and
determining a noise indicator value for each of the plurality of ECG lead signals (410) based on a correspondence between different components of each of the transformed plurality of ECG lead signals.

11. The computer-implemented method of claim 10, wherein the dimensionality reduction algorithm comprises at least one of: a singular value decomposition algorithm; and an independent component analysis algorithm.

12. The computer-implemented method of any of claims 1 to 11, wherein the method further comprises generating a display control signal (540), the display control signal indicating the identified at least one noisy electrode.

13. The computer-implemented method of any of claims 1 to 12, wherein the method further comprises analyzing a subset of the plurality of ECG lead signals to detect arrhythmia (550), the subset of the plurality of ECG lead signals consisting of the ECG lead signals which are generated by a respective pair of electrodes not including the identified at least one noisy electrode.

14. A computer program product comprising computer program code means which, when executed on a computing device having a processing system, cause the processing system to perform all of the steps of the computer-implemented method according to any of claims 1 to 13.

15. A system (600) for identifying noisy electrodes of an ECG measurement system, the system comprising:
a processing arrangement (620) configured to:
determine a noise indicator value for each of a plurality of ECG lead signals, each ECG lead signal generated by a respective pair of electrodes of an ECG measurement system during an ECG measurement of a subject;
analyze the determined noise indicator values for each of the plurality of ECG lead signals in combination with a correspondence between each ECG lead signal and its respective pair of electrodes; and
identify at least one noisy electrode based on the result of the analysis.
